# EUROPÄISCHE PATENTANMELDUNG

(11) **EP 2 359 871 A1**
(43) Veröffentlichungstag der Anmeldung: **24.08.2011**
(21) Anmeldenummer: 11002607.7
(22) Anmeldetag: 27.09.2007
(51) Int. Cl.: A61L 17/14

(54) **Chirurgisches Nahtmaterial mit antimikrobieller Oberfläche und Verfahren zur antimikrobiellen Beschichtung chirurgischen Nahtmaterials**

(30) Priorität: 25.10.2006 DE 102006051093
(62) Teilanmeldung aus: 07018993.1
(71) Anmelder: Heraeus Kulzer GmbH, 63450 Hanau (DE)
(72) Erfinder: Vogt, Sebastian, 99092 Erfurt (DE); Kühn, Klaus-Dieter, Prof. Dr., 35041 Marburg-Elnhausen (DE); Büchner, Hubert, Dr., 90491 Nürnberg (DE)
(74) Vertreter: Brand, Normen

(57) **Zusammenfassung**

Es wird ein chirurgisches Nahtmaterial mit antimikrobieller Oberfläche beschrieben, wobei die Oberfläche eine Beschichtung enthaltend
mindesten eine Fettsäure,
Octenidindichlorid und/oder Dequaliniumchlorid sowie
optional oligomere Milchsäureester aufweist. Außerdem wird ein Verfahren zur Beschichtung chirurgischen Nahtmaterials beschrieben, das dadurch charakterisiert ist, dass das Fadenmaterial mit einer homogenen methanolischen Lösung von Octenidindichlorid und/oder Dequaliniumchlorid benetzt wird und anschließend das Methanol verdampft wird, wobei sich eine Beschichtung auf der Fadenoberfläche ausbildet.

## Beschreibung

Gegenstand der Erfindung sind chirurgisches Nahtmaterial mit antimikrobieller Beschichtung und Verfahren zur antimikrobiellen Beschichtung von chirurgischem Nahtmaterial.

Chirurgisches Nahtmaterial kann man hinsichtlich seines Aufbaus in monofile und in polyfile Fäden unterteilen. Polyfile Fäden können einen so genannten Dochteffekt zeigen. Das bedeutet, durch Kapillarwirkung wandert Gewebsflüssigkeit entlang des Fadens. Damit kann auch eine Migration von mikrobiellen Keimen verbunden sein, so dass sich entlang des Fadenmaterials Infektionen ausbreiten können. Es ist daher wünschenswert, chirurgisches Nahtmaterial so auszurüsten, dass eine Keimbesiedelung auf der Fadenoberfläche und damit eine Migration von Keimen entlang des Fadens wirksam verhindert wird.

Bei den resorbierbaren Nahtmaterialien sind polyfile, geflochtene Polyglykolid-Fäden von besonderer praktischer Bedeutung. Es gibt bisher auf dem Markt nur ein von der Firma Ethicon vertriebenes, auf Polyglykolid basierendes Fadenmaterial, das mit dem Antiseptikum Triclosan ausgerüstet ist. Bei diesem Antiseptikum handelt es sich um ein gechlortes Biphenylderivat, das eine antiseptische Wirkung auf Gram-positive Bakterien ausübt. Wünschenswert wäre jedoch ein Fadenmaterial mit einer wesentlich breiteren antimikrobiellen Wirksamkeit, bei der auch Gram-negative Bakterien, Hefen und Pilze mit erfasst werden. Auch bei der Gruppe der nichtresorbierbaren Fadenmaterialien sind polyfile Fäden üblich. So wird zum Beispiel polyfiler, chirurgischer Stahldraht in der Thoraxchirurgie eingesetzt. Es sind gegenwärtig keine kommerziell vertriebenen, antimikrobiell ausgerüsteten nichtresorbierbaren Nahtmaterialien bekannt.

Es sind eine Vielzahl von antiseptisch wirksamen Substanzen seit Jahrzehnten bekannt und im medizinischen Gebrauch. Neben in Wasser löslichen Chlorhexidinsalzen in der Zahnpflege sind vor allem Octenidindichlorid-Lösungen unter dem Namen Octenisept® von der Firma Schüle & Mayr in der Chirurgie zur Wunddesinfektion und zur Flächendesinfektion weit verbreitet. Beim Octenisept handelt es sich um wässrig-alkoholische Lösungen von Octenidindichlorid und Phenoxyethanol. Das Octenidindichlorid ((1,1'-(1,10-Decandiyl)bis[4-(octylamino)pyridinium]-dichlorid) zeichnet sich gegenüber anderen kationischen Antiseptika dadurch aus, dass es zweifach positiv geladen ist. Octenidindichlorid hat ein sehr weites Wirkungsspektrum und umfasst Gram-positive und Gram-negative Bakterien als auch Hefen und Pilze. Es wurde 1977 von der Sterling Inc. entwickelt.

Eine ähnliche Struktur besitzt Dequaliniumchlorid mit ebenfalls zwei kationischen Zentren. Dequaliniumchlorid (1,10-Decamethylenbis(4-amino-2-methyl-chinolinium)-dichlorid) wurde 1957 von Allen & Hanburys beschrieben.

Sowohl Octenidindichlorid als auch Dequaliniumchlorid zeichnen sich durch ein sehr breites Wirkungsspektrum und eine gute Verträglichkeit mit Weichgewebe, insbesondere mit Schleimhaut, aus. Entsprechend wurden z.B. Octenidindichlorid-haltige Mittel zur Wund und Schleimhautdesinfektion beschrieben (DE 101 09 925 A1). Octenidindichlorid und Dequaliniumchlorid zeigen allerdings keine ausgeprägte Haftwirkung auf der Oberfläche von üblichem chirurgischem Nahtmaterial.

Der Erfindung liegt die Aufgabe zu Grunde, ein chirurgisches Nahtmaterial zu entwickeln, das so ausgerüstet ist, dass es vor einer mikrobiellen Besiedelung geschützt ist und dass der antimikrobielle Schutz ein breites Keimspektrum erfasst. Weiterhin soll ein unkompliziertes Verfahren entwickelt werden, das eine antimikrobielle Beschichtung vom kommerziellen Nahtmaterial erlaubt. Das Verfahren soll so gestaltet sein, dass das Nahtmaterial nicht durch den Beschichtungsvorgang in seiner Struktur beeinträchtigt wird.

Überraschenderweise wurde gefunden, dass durch Zusatz mindestens einer Fettsäure Octenidindichlorid und auch Dequaliniumchlorid auf den Oberflächen von Nahtmaterial zur Haftung gebracht werden können. Die Antiseptika Octenidindichlorid und Dequaliniumchlorid können zusammen mit der/den als Haftvermittler wirkenden Fettsäure/n auf die Oberfläche von chirurgischem Nahtmaterial aufgebracht werden. Es ergibt sich, dass eine Beschichtung aus einem Gemisch aus mindestens einer Fettsäure und Octenidindichlorid und/oder Dequaliniumchlorid auf der Fadenoberfläche aufgebracht ist.

Als Fettsäuren bevorzugt werden Laurinsäure, Palmitinsäure und Stearinsäure. Die Fettsäuren müssen nicht unbedingt als reine Substanzen eingesetzt werden. Es ist auch möglich, mit Monofettsäureglyceriden, Difettsäureglyceriden und Trifettsäureglyceriden verunreinigte Fettsäuren einzusetzen. Weiterhin ist es möglich, Gemische aus Fettsäuren und oligomeren Milchsäure-estern zu verwenden.

Das Gewichtsverhältnis von Octenidindichlorid oder Dequaliniumchlorid zur Fettsäure beträgt vorzugsweise 1 zu 0,1 bis 1 zu 5 ist, besonders bevorzugt 1 zu 1.

Die Aufgabe der Erfindung wird auch durch ein Verfahren zur Beschichtung chirurgischen Nahtmaterials gelöst; bei dem das Material mit einer homogenen methanolischen Lösung von Octenidindichlorid und/oder Dequaliniumchlorid und mindestens einer Fettsäure benetzt wird und anschließend das Methanol verdampft wird, wobei sich eine Beschichtung auf der Fadenoberfläche ausbildet. Es ist auch möglich, anstelle von Methanol Gemische aus Methanol und anderen niederen Alkoholen, wie Ethanol, Propanol und Isopropanol, einzusetzen. Der Vorteil der Verwendung von methanolischen Lösungen besteht auch darin, dass übliche Polyglykolid-, Polydioxanon- und Poly-epsilon-caprolacton-co-L-lactid-Fäden durch kurzzeitige Einwirkung von Methanol nicht angequollen werden und damit die Fadenstruktur erhalten bleibt.

Die Erfindung wird durch die nachstehenden Beispiele näher erläutert, ohne jedoch die Erfindung zu beschränken.

### Beispiel 1:

In 28,800 g Methanol (Fluka) werden 300 mg Dequaliniumchlorid (Solmag) und 300 mg Palmitinsäure (Fluka) bei Raumtemperatur gelöst. Es entsteht eine klare Lösung. In diese Lösung wird ein 50 cm langes Stück eines geflochtenen Polyglycolid-Fadens (USP 2.0) getaucht. Anschließend lässt man das Methanol bei Raumtemperatur verdampfen. Die Masse der Beschichtung wird gravimetrisch erfasst. Die Masse der Beschichtung beträgt 0,6 mg.

### Beispiel 2:

In 28,800 g Methanol (Fluka) werden 600 mg Octenidindichlorid (Dishman Pharmaceuticals) und 600 mg Palmitinsäure (Fluka) bei Raumtemperatur gelöst. Es entsteht eine klare Lösung. In diese Lösung wird ein 50 cm langes Stück eines geflochtenen Polyglycolid-Fadens (USP 2.0) getaucht. Anschließend lässt man das Methanol bei Raumtemperatur verdampfen. Die Masse der Beschichtung wird gravimetrisch erfasst. Die Masse der Beschichtung beträgt 1,4 mg.

## Patentansprüche

1. Chirurgisches Nahtmaterial mit antimikrobieller Oberfläche, **dadurch gekennzeichnet, dass** die Oberfläche eine Beschichtung enthaltend
• mindestens eine Fettsäure,
• Octenidindichlorid und/oder Dequaliniumchlorid und
• optional oligomere Milchsäureester
aufweist.

2. Chirurgisches Nahtmaterial nach Anspruch 1, **dadurch gekennzeichnet, dass** die mindestens eine Fettsäure der Gruppe bestehend aus Laurinsäure, Palmitinsäure und Stearinsäure angehört.

3. Chirurgisches Nahtmaterial nach Anspruch 1, **dadurch gekennzeichnet, dass** das Gewichtsverhältnis von Octenidindichlorid oder Dequaliniumchlorid zur Fettsäure oder der Summe der Fettsäuren 1,0 zu 0,1 bis 1,0 zu 5,0 beträgt

4. Chirurgisches Nahtmaterial nach Anspruch 3, wobei das Gewichtsverhältnis 1,0 zu 1,0 beträgt.

5. Verfahren zur Beschichtung chirurgischen Nahtmaterials mit den Schritten
• Benetzung des Fadenmaterials mit einer homogenen methanolischen Lösung von Octenidindichlorid und/oder Dequaliniumchlorid und mindestens einer Fettsäure,
• anschließendes Verdampfen des Methanols.

6. Verfahren nach Anspruch 5, wobei die methanolische Lösung von der Gruppe der Gemische aus Methanol und anderen niederen Alkoholen gebildet wird.

7. Verfahren nach Anspruch 6, wobei die Alkohole der Gruppe Ethanol, Propanol und Isopropanol angehören.
